# EUROPÄISCHE PATENTANMELDUNG

(11) **EP 3 037 125 A1**
(43) Veröffentlichungstag der Anmeldung: **29.06.2016**
(21) Anmeldenummer: 14200214.6
(22) Anmeldetag: 23.12.2014
(51) Int. Cl.: A61M 37/00, A01K 11/00

(54) **Handgerät zum lokalen Aufstechen einer menschlichen oder einer tierischen Haut und Verfahren zum Betrieb**

(71) Anmelder: MT.DERM GmbH, 14167 Berlin (DE)
(72) Erfinder: Scherkowski, Dirk, 12489 Berlin (DE); Hoes, Jean Armand Jacques André, 6001CM Weert (NL)
(74) Vertreter: Bittner, Thomas L.

(57) **Zusammenfassung**

Die Erfindung betrifft ein Handgerät zum lokalen Aufstechen einer menschlichen oder einer tierischen Haut, in einem Gehäuse (1) aufweisend: eine Antriebseinrichtung (2), die eingerichtet ist, eine Antriebskraft bereitzustellen; eine Stecheinrichtung, bei der ein Stechwerkzeug (7) an einer verlagerbaren Werkzeugaufnahme (8) angeordnet ist, wobei die Werkzeugaufnahme mit dem Stechwerkzeug in Bezug auf eine Stechwerkzeugöffnung (9) an dem Gehäuse zwischen einer eingefahrenen und einer ausgefahrenen Stellung verlagerbar ist und hierbei in einer Längsrichtung bewegt wird; und eine Kopplungseinrichtung (6), die an die Antriebseinrichtung und die Stecheinrichtung koppelt und die Antriebskraft auf die Stecheinrichtung überträgt, wobei die Stecheinrichtung eingerichtet ist, während einer Verlagerung der Werkzeugaufnahme mit dem Stechwerkzeug zwischen der eingefahrenen und der ausgefahrenen Stellung eine durch die Antriebskraft und / oder eine weitere Antriebskraft, die im Gehäuse aktiv oder passiv erzeugt wird, erzwungene Verlagerung des Stechwerkzeugs relativ zu der Längsrichtung zuzulassen. Weiterhin ist ein Verfahren zum Betrieb des Handgeräts geschaffen.

## Beschreibung

Die Erfindung betrifft ein Handgerät zum lokalen Aufstechen einer menschlichen oder einer tierischen Haut sowie ein Verfahren zum Betrieb des Handgeräts.

### Hintergrund

Solche Handgeräte werden benutzt, um eine menschliche oder eine tierische Haut lokal aufzustechen und hierbei eine Substanz in die Haut einzutragen, zum Beispiel in Verbindung mit dem Herstellen eines Tattoos oder von permanentem Makeup. Aber auch das Eintragen von medizinischen oder kosmetischen Wirkstoffen ist auf diese Weise möglich. Regelmäßig sind die Handgeräte ausgebildet, die Stechbewegung repetitiv mit einer Wiederholfrequenz auszuführen, so dass die Haut wiederholt aufgestochen wird zum Wirkstoff- oder Substanzeintrag.

Bei den bekannten Handgeräten ist in einem Gehäuse eine Antriebseinrichtung angeordnet, die eingerichtet ist, eine Antriebskraft repetitiv mit einer Wiederholfrequenz bereitzustellen. Hierbei können beispielsweise Elektromotoren eingesetzt werden, die eine drehende Antriebsbewegung zur Verfügung stellen, welche anschließend mittels eines Wandlungsmechanismus in eine lineare Vor- und Zurückbewegung in Längsrichtung des Gehäuses gewandelt wird. Derartige Antriebseinrichtungen sind als solche in verschiedenen Ausführungsformen bekannt. Die so erzeugte Antriebskraft wird auf eine in dem Gehäuse angeordnete Stecheinrichtung gegeben, welche ein Stechwerkzeug zum Aufstechen der Haut aufweist, das seinerseits an einer Werkzeugaufnahme angeordnet ist. Beispielsweise kann es sich um eine Stechnadel handeln, die an einem Nadelschaft angeordnet ist. Im Betrieb wird das Stechwerkzeug wiederholt vor- und zurückgefahren, um so bei der Verwendung des Handgeräts die Haut mehrfach lokal aufzustechen. Antriebseinrichtung und Stecheinrichtung sind über eine Kopplungseinrichtung funktionell miteinander verbunden, so dass die repetitive Antriebskraft auf die Stecheinrichtung eingekoppelt werden kann. Das Stechwerkzeug führt eine Vor- und Rückwärtsbewegung auf einer linearen Bewegungsbahn aus, die üblicherweise parallel zur Längsrichtung des Gehäuses des Handgeräts verläuft.

An oder in dem Gehäuse kann ein Reservoir vorgesehen sein, welches mit der in die Haut einzubringenden Substanz befüllt ist, um im Betrieb dem distalen Ende des Stechwerkzeugs die einzubringende Substanz zuzuführen, zum Beispiel aufgrund von Kapillarkräften, die im Bereich der Oberfläche des Stechwerkzeugs wirken.

### Zusammenfassung

Aufgabe der Erfindung ist es, ein Handgerät zum lokalen Aufstechen einer menschlichen oder einer tierischen Haut sowie ein Verfahren zum Betrieb des Handgeräts anzugeben, die die Nutzungsmöglichkeiten derartiger Geräte erweitern, insbesondere einen verbesserten Substanzeintrag in die Haut ermöglichen.

Zur Lösung der Aufgabe ist ein Handgerät zum lokalen Aufstechen einer menschlichen oder einer tierischen Haut nach dem unabhängigen Anspruch 1 geschaffen. Weiterhin ist ein Verfahren zum Betrieb des Handgeräts nach dem unabhängigen Anspruch 13 vorgesehen. Ausgestaltungen sind Gegenstand von abhängigen Unteransprüchen.

Nach einem Aspekt ist ein Handgerät zum lokalen Aufstechen einer menschlichen oder einer tierischen Haut geschaffen, bei dem in einem Gehäuse angeordnet sind
- eine Antriebseinrichtung, die eingerichtet ist, eine Antriebskraft bereitzustellen,
- eine Stecheinrichtung, bei der ein Stechwerkzeug an einer verlagerbaren Werkzeugaufnahme angeordnet ist, wobei die Werkzeugaufnahme mit dem Stechwerkzeug in Bezug auf eine Stechwerkzeugöffnung an dem Gehäuse zwischen einer eingefahrenen und einer ausgefahrenen Stellung verlagerbar ist und hierbei in einer Längsrichtung bewegt wird, und
- eine Kopplungseinrichtung, die an die Antriebseinrichtung und die Stecheinrichtung koppelt und die Antriebskraft auf die Stecheinrichtung überträgt,
wobei die Stecheinrichtung eingerichtet ist, während einer Verlagerung der Werkzeugaufnahme mit dem Stechwerkzeug zwischen der eingefahrenen und der ausgefahrenen Stellung eine durch die Antriebskraft und / oder eine weitere Antriebskraft, die im Gehäuse aktiv oder passiv erzeugt wird, erzwungene Verlagerung des Stechwerkzeugs relativ zu der Längsrichtung zuzulassen.

Nach einem weiteren Aspekt ist ein Verfahren zum Betrieb eines Handgeräts geschaffen, wobei das Verfahren die folgenden Schritte aufweist:
- Erzeugen einer Antriebskraft mittels einer Antriebseinrichtung, die in einem Gehäuse angeordnet ist, und
- Übertragen der Antriebskraft mittels einer Kopplungseinrichtung von der Antriebseinrichtung auf eine Stecheinrichtung, bei der ein Stechwerkzeug an einer verlagerbaren Werkzeugaufnahme angeordnet ist, derart, dass die Werkzeugaufnahme mit dem Stechwerkzeug in Bezug auf eine Stechwerkzeugöffnung an dem Gehäuse zwischen einer eingefahrenen und einer ausgefahrenen Stellung verlagert wird und hierbei in einer Längsrichtung bewegt wird,
wobei während einer Verlagerung der Werkzeugaufnahme mit dem Stechwerkzeug zwischen der eingefahrenen und der ausgefahrenen Stellung das Stechwerkzeug eine erzwungene Verlagerung relativ zu der Längsrichtung ausführt, wobei die Relativverlagerung durch die Antriebskraft und / oder eine weitere Antriebskraft erzwungen wird, die im Gehäuse aktiv oder passiv erzeugt wird.

Das Stechwerkzeug führt im Betrieb aktiv und / oder passiv angetrieben eine ergänzende Verlagerung aus, die zusätzlich zu der linearen Verlagerung in Längsrichtung zwischen der eingefahrenen und der ausgefahrenen Stellung stattfindet. Die zusätzliche Verlagerung des Stechwerkzeugs kann beim Ausfahren und / oder beim Einfahren erzwungen werden. Alternativ oder ergänzend kann die zusätzliche Verlagerung des Stechwerkzeugs erzwungen werden, wenn das Stechwerkzeug momentan in einer Stellung ruht, zum Beispiel in einer vollständig ausgefahrenen Stellung (distale Endstellung). Wird das Handgerät zum Aufstechen der Haut angewendet, kann die zusätzliche Verlagerung des Stechwerkzeugs zum Beispiel zum zeitweiligen Aufweiten der in der Haut mittels Aufstechen erzeugten Öffnung führen, so dass die in die Haut einzubringende Substanz besser eindringen kann. Im Betrieb des Handgeräts überlagern sich die lineare Vor- und Zurückbewegung in Längsrichtung sowie die zusätzlich stattfindende Verlagerung des Stechwerkzeugs in Relation zur Längsrichtung.

Zum Erzwingen der zusätzlichen Verlagerung kann ein Bewegungsmechanismus vorgesehen sein, welcher eine aktiv mittels eines Aktors und / oder eine passiv erzeugte Bewegungskraft auf die Werkzeugaufnahme und / oder das stechwerkzeug einkoppelt. Die Bewegungskraft als zum Beispiel als lineare Antriebskraft und / oder drehende Antriebskraft eingekoppelt werden.

Es kann ein Handgerät zum lokalen Aufstechen einer menschlichen oder einer tierischen Haut vorgesehen sein, bei dem in einem Gehäuse eine Antriebseinrichtung angeordnet ist, die eingerichtet ist, eine Antriebskraft bereitzustellen. In dem Gehäuse sind weiterhin eine Stecheinrichtung und eine Kopplungseinrichtung angeordnet. Bei der Stecheinrichtung ist ein Stechwerkzeug an einer verlagerbaren Werkzeugaufnahme angeordnet, wobei die Werkzeugaufnahme mit dem Stechwerkzeug in Bezug auf eine Stechwerkzeugöffnung an dem Gehäuse zwischen einer eingefahrenen und einer ausgefahrenen Stellung verlagerbar ist und hierbei in einer Längsrichtung bewegt wird. Das Stechwerkzeug bewegt sich vor und zurück. Zumindest in der ausgefahrenen Stellung kann ein distales Ende des Stechwerkzeugs mit einer Stechwerkzeugspitze außerhalb des Gehäuses angeordnet und der Stechwerkzeugöffnung vorgelagert sein. Die Antriebseinrichtung und die Stecheinrichtung koppeln an die Kopplungseinrichtung, mit der die Antriebskraft zum Aus- und Einfahren von der Antriebseinrichtung auf die Stecheinrichtung übertragen wird. Im Gehäuse des Handgeräts kann weiterhin ein Querbewegungsmechanismus vorgesehen sein, der an die Stecheinrichtung koppelt und eingerichtet ist, eine Querbewegungskraft quer zur Längsrichtung der Verlagerung der Werkzeugaufnahme mit dem Stechwerkzeug zwischen der eingefahrenen und der ausgefahrenen Stellung und diese im Betrieb auf die Stecheinrichtung einzukoppeln.

Weiterhin kann ein Verfahren zum Betreiben eines Handgeräts vorgesehen sein, welches die folgenden Schritte aufweist: Erzeugen einer Antriebskraft mittels einer Antriebseinrichtung, die in einem Gehäuse angeordnet ist, und Übertragen der Antriebskraft mittels einer Kopplungseinrichtung von der Antriebseinrichtung auf eine Stecheinrichtung, bei der ein Stechwerkzeug an einer verlagerbaren Werkzeugaufnahme angeordnet ist, derart, dass die Werkzeugaufnahme mit dem Stechwerkzeug in Bezug auf eine Stechwerkzeugöffnung an dem Gehäuse zwischen einer eingefahrenen und einer ausgefahrenen Stellung verlagert wird und hierbei in einer Längsrichtung bewegt wird. Mittels eines Querbewegungsmechanismus, der an die Stecheinrichtung koppelnd in dem Gehäuse angeordnet ist, wird eine Querbewegungskraft quer zu der Längsrichtung der Verlagerung der Werkzeugaufnahme mit dem Stechwerkzeug zwischen der eingefahrenen und der ausgefahrenen Stellung erzeugt und auf die Stecheinrichtung eingekoppelt.

Unter dem Einfluss der Querbewegungskraft, welche von dem Querbewegungsmechanismus bereitgestellt wird, führt das Stechwerkzeug im Betrieb eine ergänzende Verlagerung aus, die zusätzlich zu der linearen Verlagerung zwischen der eingefahrenen und der ausgefahrenen Stellung stattfindet. Wird das Handgerät zum Aufstechen der Haut angewendet, kann eine zusätzliche Seitwärtsverlagerung des Stechwerkzeugs zum Beispiel zum vorübergehenden Aufweiten der in der Haut mittels Aufstechen erzeugten Öffnung führen, so dass die in die Haut einzubringende Substanz besser eindringen kann. Im Betrieb des Handgeräts überlagern sich die lineare Vor- und Zurückbewegung in Längsrichtung sowie die aufgrund der Querbewegungskraft zusätzlich stattfindende Verlagerung der Werkzeugaufnahme mit dem Stechwerkzeug.

Das Einkoppeln der Querbewegungskraft kann stattfinden, während sich die Werkzeugaufnahme mit dem Stechwerkzeug bewegt und / oder in einer momentanen Ruhestellung der Werkzeugaufnahme mit Stechwerkzeug, zum Beispiel in der am weitesten ausgefahrenen Stellung (distale Endstellung). Das Einkoppeln der Querbewegungskraft kann im Verlauf einer Aus- und Einfahrbewegung einmalig oder in zeitlichen Abständen mehrfach erfolgen.

Die Bewegungskraft, insbesondere die Querbewegungskraft, kann in einem Winkel von etwa 90° Grad zur Längsrichtung eingekoppelt werden.

Vor- und Zurückbewegung kann in den verschiedenen Ausgestaltungen mittels der Antriebseinrichtung als Einzelhub / Einzelstechbewegung oder repetitiv mit einer Wiederholfrequenz ausgeführt werden. Im Fall der repetitiven Bewegung kann die Wiederholfrequenz wenigstens etwa 0,1Hz betragen. Alternativ kann die Wiederholfrequenz wenigstens etwa 10Hz betragen. Bei einer weiteren alternativen Ausführung kann die Wiederholfrequenz wenigstens etwa 50Hz betragen. Eine Obergrenze der Wiederholfrequenz kann etwa 100Hz betragen, bei einer Alternative etwa 150Hz.

Das Stechwerkzeug kann mit einer oder mehreren Stechnadeln oder einer Nadelplatte gebildet sein. Mehrere Stechnadeln können eine Nadelgruppe bilden. Im Fall der Verwendung einer oder mehrere Nadeln kann die Stechwerkzeugöffnung auch als Nadelöffnung des Gehäuses bezeichnet werden. Die Stechwerkzeugöffnung kann in einem Stechwerkzeugmodul gebildet sein, welches lösbar an einem Antriebsmodul angeordnet sein kann, welches seinerseits die Antriebseinrichtung aufnimmt. Ein solches Stechwerkzeugmodul kann als Einwegmodul ausgeführt sein.

Die Werkzeugaufnahme, an welcher das Stechwerkzeug angeordnet ist, kann ein- oder mehrstückig ausgeführt sein.

Im Fall der Verwendung einer oder mehrerer Stechnadeln als Stechwerkzeug kann die Werkzeugaufnahme als Nadelschaft ausgebildet sein.

Es kann vorgesehen sein, dass der Bewegungsmechanismus, insbesondere der Querbewegungsmechanismus, direkt an die Werkzeugaufnahme koppelt und hierdurch die Bewegungskraft, insbesondere die Querbewegungskraft, wenigstens teilweise direkt in die Werkzeugaufnahme einkoppelbar ist. Der Bewegungsmechanismus koppelt hierbei nicht indirekt, zum Beispiel über das Stechwerkzeug, an die Werkzeugaufnahme, sondern mittels direkter Kopplung.

Es kann weiterhin vorgesehen sein, dass der Bewegungsmechanismus, insbesondere der Querbewegungsmechanismus, direkt an das Stechwerkzeug koppelt und hierdurch die Bewegungskraft, insbesondere die Querbewegungskraft, wenigstens teilweise direkt in das Stechwerkzeug einkoppelbar ist. Der Bewegungsmechanismus koppelt hierbei nicht indirekt, zum Beispiel über die Werkzeugaufnahme, an das Stechwerkzeug, sondern mittels direkter Kopplung. Zum Beispiel kann ein Arbeitsstößel gegen einen Abschnitt des Stechwerkzeugs drücken. In ähnlicher Weise kann eine direkte Kopplung des Bewegungsmechanismus an die Werkzeugaufnahme erfolgen. Der Querbewegungsmechanismus kann eine aktive Querbewegungseinrichtung aufweisen, bei der die Querbewegungskraft aktiv mittels der Antriebseinrichtung und / oder einer weiteren Antriebseinrichtung erzeugt wird, die in dem Gehäuse angeordnet ist. Bei dieser Ausführungsform erfolgt eine aktive Erzeugung der Querbewegungskraft mit Hilfe eines Antriebs (Aktors), bei dem es sich um die Antriebseinrichtung oder eine weitere Antriebseinrichtung in dem Gehäuse handeln kann. Zum Beispiel kann die Einleitung der aktiv erzeugten Antriebskraft über ein Gelenk erfolgen. Die mit Hilfe der weiteren Antriebseinrichtung erzeugte Antriebskraft, insbesondere für die Querbewegung, kann repetitiv erzeugt werden, insbesondere mit gleicher Wiederholfrequenz wie die Antriebskraft für die Verlagerung der Werkzeugaufnahme mit Stechwerkzeug zwischen der eingefahrenen und der ausgefahrenen Stellung oder mit einem Vielfachen dieser Wiederholfrequenz. Die Antriebseinrichtung und / oder die weitere Antriebseinrichtung können einen elektrischen, einen pneumatischen oder einen hydraulischen Antrieb (Aktor) aufweisen, mit denen eine jeweilige Antriebskraft aktiv erzeugt wird. Es kann eine lineare Antriebskraft bereitgestellt werden. Auch das Bereitstellen einer drehenden Antriebsbewegung kann bei der Antriebseinrichtung und / oder der weiteren Antriebseinrichtung vorgesehen sein, wobei die drehende Antriebsbewegung mit Hilfe eines Wandlungsmechanismus in eine lineare Antriebsbewegung wandelbar sein kann. Teile des Wandlungsmechanismus können Bestandteil der Kopplungseinrichtung sein, die die erzeugte Antriebskraft auf die Stecheinrichtung überträgt. In Verbindung mit den Antrieben kann vorgesehen sein, dass sich hierbei ein Arbeitskolben in einem Kolbengehäuse bewegt.

Der Querbewegungsmechanismus kann eine passive Querbewegungseinrichtung aufweisen, bei der die Querbewegungskraft frei von einer Antriebseinrichtung passiv erzeugt wird. Es kann ein passiver Verschiebemechanismus vorgesehen sein, zum Beispiel in Form einer passiven Bauteilführung. Die passive Querbewegungseinrichtung ist frei von einem eigenen Antrieb (aktorfrei). Die Querbewegungskraft wird beispielsweise erzeugt, indem ein an die Werkzeugaufnahme und / oder das Stechwerkzeug koppelndes Bauteil auf einer Rampe bewegt wird, die zur Längsrichtung der Verlagerungsbewegung schräggestellt ist.

In vergleichbarer Weise kann der Bewegungsmechanismus für eine aktive oder eine passive Erzeugung der Bewegungskraft ausgestaltet sein.

Das Stechwerkzeug kann schwenkbar sein. Das Stechwerkzeug kann schwenkbar an der Werkzeugaufnahme angeordnet sein. Bei einer alternativen Ausführungsform ist das Stechwerkzeug zusammen mit der Werkzeugaufnahme schwenkbar gelagert. Die Schwenkbarkeit kann beispielsweise unter Verwendung eines Kugelgelenks bereitgestellt sein. Das Schwenken des Stechwerkzeugs kann um eine Achse ermöglicht sein, die in der Längsrichtung verläuft. Das Schwenken des Stechwerkzeugs kann kombiniert sein mit einem Kippen des Stechwerkzeugs um einen Auflagerpunkt, der beispielsweise am Gehäuse des Handgeräts gebildet ist, zum Beispiel im Bereich der Stechwerkzeugöffnung.

Die Werkzeugaufnahme kann in dem Gehäuse mittels einer Führung geführt sein, die Führungsachsen in Längsrichtung und quer hierzu aufweisen. Die Werkzeugaufnahme kann in der Führung gelagert sein oder über eine hiermit verbundene Lagerung hieran koppeln. Zum Beispiel kann eine Schlitzführung vorgesehen sein.

Mittels einer Vorspanneinrichtung kann die Werkzeugaufnahme mit dem Stechwerkzeug gegen das Verlagern in die ausgefahrene Stellung und / oder das Verlagern in die eingefahrene Stellung vorgespannt sein. Eine weitere Vorspanneinrichtung kann die Werkzeugaufnahme mit dem Stechwerkzeug gegen die Relativverlagerung vorspannen, insbesondere gegen die Querbewegung. Eine Vorspannkraft kann zum Beispiel mit Hilfe eines Federmechanismus bereitgestellt sein.

Die Werkzeugaufnahme kann mit einem Gelenkmechanismus verbunden sein, welcher über ein aufnahmeseitiges Gelenkelement an die Werkzeugaufnahme koppelt und bei dem das aufnahmeseitige Gelenkelement an ein oder mehrere weitere Gelenkelemente gelenkig koppelt, die an einem oder mehreren zugeordneten Gelenklagerungspunkten gelagert sind. Der Gelenkmechanismus kann mit einem Kurbelgelenk gebildet sein.

Die Werkzeugaufnahme kann mit einer im Gehäuse aufgenommenen Dämpfungseinrichtung verbunden sein. Zum Beispiel kann ein Federdämpfungsmechanismus vorgesehen sein.

Die Relativverlagerung des Stechwerkzeugs kann stattfinden aufgrund Verschieben, Verschwenken und / oder Verkippen des Stechwerkzeugs, insbesondere infolge des Einkoppelns der Querbewegungskraft.

Die Gehäuseöffnung kann eine sich quer zu der Längsrichtung erstreckenden Öffnungsführung für das Stechwerkzeug aufweisen, in welcher das Stechwerkzeug bei der Verlagerung relativ zur Längsrichtung verlagert wird, insbesondere infolge des Einkoppelns der Querbewegungskraft.

In Verbindung mit dem Verfahren zum Betreiben des Handgeräts können die vorangehend im Zusammenhang mit dem Handgerät beschriebenen Ausgestaltungen entsprechend vorgesehen sein.

### Beschreibung von Ausführungsbeispielen

Im Folgenden werden weitere Ausführungsbeispiele unter Bezugnahme auf Figuren einer Zeichnung näher erläutert. Hierbei zeigen:
- Fig. 1: eine schematische Darstellung eines Handgeräts zum lokalen Aufstechen einer menschlichen oder einer tierischen Haut,
- Fig. 2: eine schematische Darstellung zur Erläuterung eines Antriebsmechanismus für das Handgerät nach Fig. 1,
- Fig. 3: eine schematische Darstellung eines weiteren Antriebsmechanismus für das Handgerät nach Fig. 1,
- Fig. 4: eine schematische Darstellung eines anderen Antriebsmechanismus für das Handgerät nach Fig. 1
- Fig. 5: eine schematische Darstellung eines Antriebsmechanismus mit passiver Querbewegungseinrichtung,
- Fig. 6: eine schematische Darstellung eines weiteren Antriebsmechanismus mit Querbewegungseinrichtung,
- Fig. 7: eine schematische Darstellung eines anderen Antriebsmechanismus mit einem Gelenkmechanismus und
- Fig. 8: eine schematische Darstellung von eines weiteren Antriebsmechanismus mit einer Gelenkanordnung.

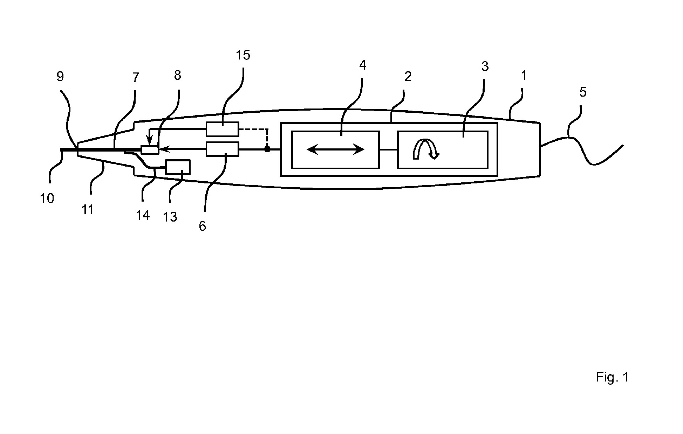

Fig. 1 zeigt eine schematische Darstellung eines Handgeräts zum lokalen Aufstechen einer menschlichen oder einer tierischen Haut mit einem Gehäuse 1. In dem Gehäuse 1 ist eine Antriebseinrichtung 2 vorgesehen, die in der dargestellten Ausführungsform einen Motor 3 sowie einen Wandlungsmechanismus 4 aufweist. Mittels des Motors 3 wird eine drehende Antriebsbewegung (drehende Antriebskraft) bereitgestellt, die mit Hilfe des Wandlungsmechanismus 4 in eine lineare Antriebskraft in Längsrichtung des Gehäuses 1 gewandelt wird, sei es zum Ausführen eines Einzelhubs oder für eine repetitive Bewegung. Derartige Wandlungsmechanismen sind als solche in verschiedenen Ausführungsformen bekannt, weshalb dies hier keiner weiteren Erläuterung bedarf. Zum Beispiel kann ein Mechanismus mit einer Taumelscheibe und einem Pleuel vorgesehen sein. Aber auch andere Wandlungsmechanismen sind als solche bekannt.

Zur Energieversorgung ist das Handgerät über eine Zuleitung 5 an eine Versorgungsspannung angeschlossen. Die Zuleitung 5 kann ergänzend Kabel umfassen, über die Steuersignale zwischen einem Steuergerät (nicht dargestellt) und dem Handgerät übertragen werden.

Die von der Antriebseinrichtung 2 bereitgestellte Antriebskraft wird über eine Koppelungseinrichtung 6 auf ein bei der dargestellten Ausführungsform als Nadel ausgeführtes Stechwerkzeug 7 gegeben, welches in einer Werkzeugaufnahme 8 angeordnet ist. Im Fall der Verwendung einer oder mehrerer Nadeln als Stechwerkzeug 7 kann es sich bei der Werkzeugaufnahme 8 um einen Nadelschaft handeln, der in dem Gehäuse 1 in einer zugeordneten Führung angeordnet ist, so dass die repetitive lineare Antriebskraft für eine Vor- und Rückwärtsbewegung von Werkzeugaufnahme 8 mit Stechwerkzeug 7 umgesetzt werden kann. Das Stechwerkzeug 7 ist durch eine Stechwerkzeugöffnung 9 an dem Gehäuse 1 geführt, so dass zumindest in einer ausgefahrenen Stellung eine Stechwerkzeugspitze 10 außerhalb des Gehäuses 1 angeordnet ist, um die zu bearbeitende Haut aufzustechen. Die Stechwerkzeugöffnung 9 ist bei der gezeigten Ausführungsform am vorderen Ende eines Stechwerkzeugmoduls 11 gebildet, welches lösbar an dem Gehäuse 1 aufgenommen sein kann.

Bei der gezeigten Ausführungsform des Handgeräts ist in dem Gehäuse 1 weiterhin ein Reservoir 13 vorgesehen, welches zur Aufnahme einer in die Haut einzubringenden Substanz dient, zum Beispiel einer Farbe im Fall des Herstellens eines Tattoos oder von permanentem Makeup. Über einen Ausfluss 14 wird die Substanz an das Stechwerkzeug 7 herangeführt, um so an der Stechwerkzeugspitze 10 die Substanz zum Einbringen in die Haut bereitzustellen. Alternativ kann das Reservoir auch außerhalb des Gehäuses 2 angeordnet sein. Verschiedene Ausgestaltungen für ein solches Reservoir und das Bereitstellen der einzubringenden Substanz im Bereich des Stechwerkzeugs 7 sind als solche bekannt, beispielsweise auch unter Verwendung einer Pumpeinrichtung an dem Reservoir 13.

Ergänzend wird bei dem Handgerät in Fig. 1 auf die Stechwerkzeugaufnahme 8 eine Querbewegungskraft eingekoppelt, die mit Hilfe eines Querbewegungsmechanismus 15 bereitgestellt ist. Mit Hilfe des Querbewegungsmechanismus 15 wird eine Querkraft bereitgestellt und auf die Stechwerkzeugaufnahme 8 und somit auf das Stechwerkzeug 7 eingekoppelt, was einer Verlagerung des Stechwerkzeugs 7 quer zur Verlagerung beim Aus- und Einfahren bewirkt, so dass Stechwerkzeug 7 quer verlagert wird und / oder verkippt wird. Die Krafteinleitung kann alternativ auch direkt auf das Stechwerkzeug 7 erfolgen.

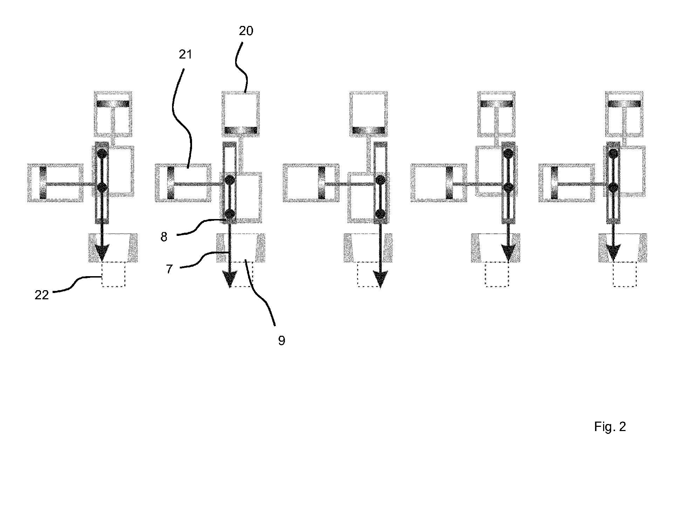

Die Fig. 2 bis 8 zeigen schematische Darstellungen für Ausführungsformen eines Antriebsmechanismus, welcher in Verbindung mit dem Handgerät zum lokalen Aufstechen einer menschlichen oder einer tierischen Haut für die Stecheinrichtung mit Stechwerkzeug 7 und Stechwerkzeugaufnahme 8 sowohl eine Linearbewegung zum Aus- und Einfahren wie auch eine Querkraft zum seitlichen Verschieben, Schwenken und / oder Verkippen des Stechwerkzeugs 7 bereitstellt. Für gleiche Merkmale werden in den Fig. 2 bis 8 gleiche Bezugszeichen verwendet.

Fig. 2 zeigt eine Ausführungsform, bei der eine Antriebseinrichtung 20 und eine weitere Antriebseinrichtung 21 vorgesehen sind, die eine jeweilige Antriebskraft bereitstellen, um das Stechwerkzeug 7 in Bezug auf die Stechwerkzeugöffnung 9 sowohl in Längsrichtung wie auch quer hierzu zu bewegen. Die Stechwerkzeugöffnung 9 ist rückseitig konisch ausgebildet.

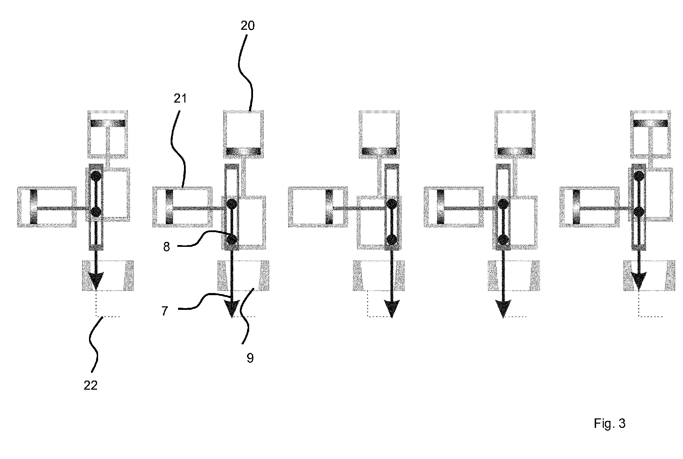

Fig. 3 zeigt eine schematische Darstellung, bei der der Antriebsmechanismus ebenfalls mit der Antriebseinrichtung 20 sowie der weiteren Antriebseinrichtung 21 gebildet ist, wobei für das Stechwerkzeug 7 ein im Vergleich zu Fig. 2 geänderter Bewegungsablauf realisiert ist, was mittels der gestrichelten Linie 22 gezeigt ist.

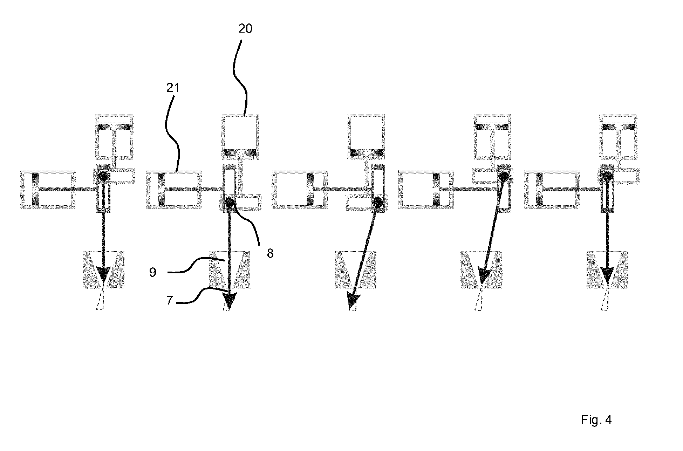

Auch bei der Ausgestaltung des Antriebsmechanismus in Fig. 4 werden die Linearbewegung zum Ein- und Ausfahren des Stechwerkzeugs 7 aktiv mittels der Antriebseinrichtung 20 sowie die Querbewegungskraft mittels des weiteren Antriebs 21 erzeugt, wobei bei dieser Ausgestaltung das Stechwerkzeug 7 aufgrund des Einkoppelns der Querbewegungskraft verschwenkt (gekippt) wird.

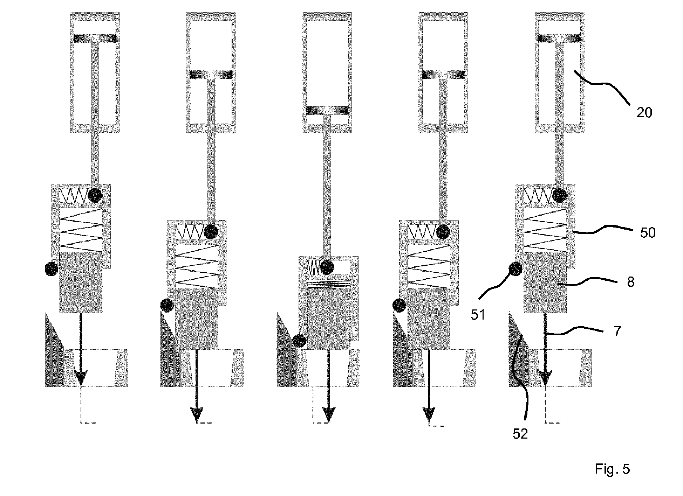

Fig. 5 zeigt eine Ausführungsform des Antriebsmechanismus für das Handgerät, bei dem die Querbewegungskraft mit Hilfe einer passiven Querbewegungseinrichtung bereitgestellt ist. Die Stechwerkzeugaufnahme 8 ist in einem Bauteil 50 aufgenommen, welches über eine Rolle 51 auf einer Rampe 52 abrollt und hierdurch quer zur Längsrichtung verlagert wird.

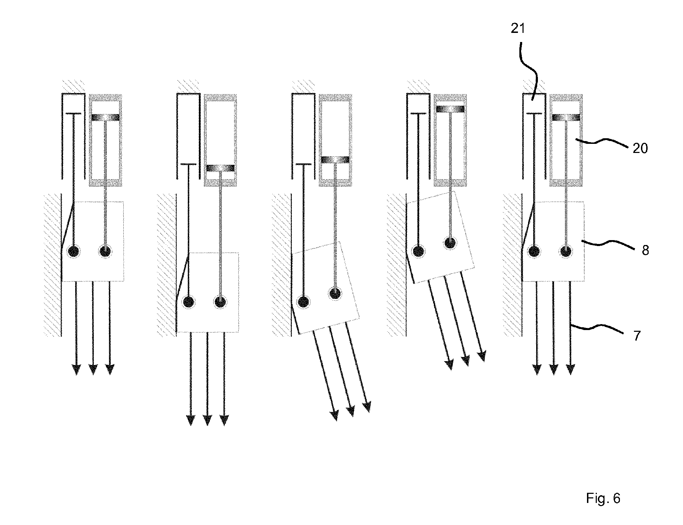

Bei der Ausführungsform in Fig. 6 sind die Antriebseinrichtung 20 und die weitere Antriebseinrichtung 21 vorgesehen, wobei die Stechwerkzeugaufnahme 8, die bei der gezeigten Ausführungsform mehrere Stechwerkzeuge 7 aufnimmt, mit Hilfe des weiteren Antriebs 21 verkippt wird. Alternativ kann anstelle der weiteren Antriebseinrichtung 21 ein richtungsunabhängiger Dämpfer zum Einsatz kommen.

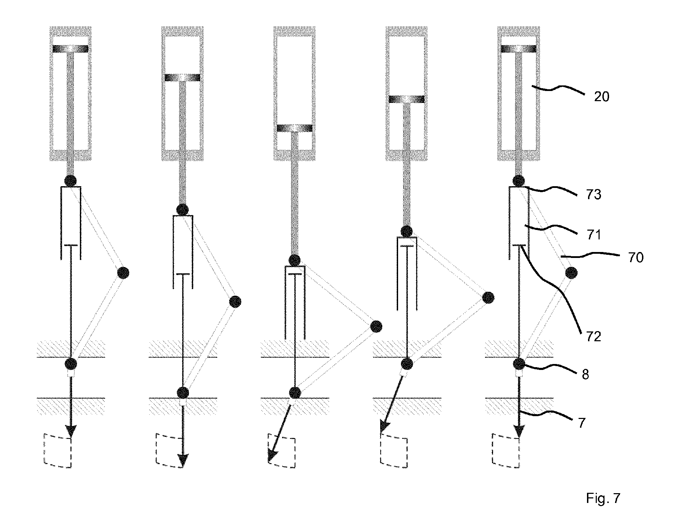

Fig. 7 zeigt eine Ausführungsform, bei der Stechwerkzeugaufnahme 8 über eine Gelenkmechanismus 70 an die Antriebseinrichtung 20 koppelt. Ein längenveränderliches Bauteil 71 ist mit einem Kolben 72 gebildet, der in einem Kolbengehäuse 73 verlagerbar angeordnet ist. Hiermit kann ein passiver Widerstand gegen das Ein- und Ausfahren bereitgestellt werden.

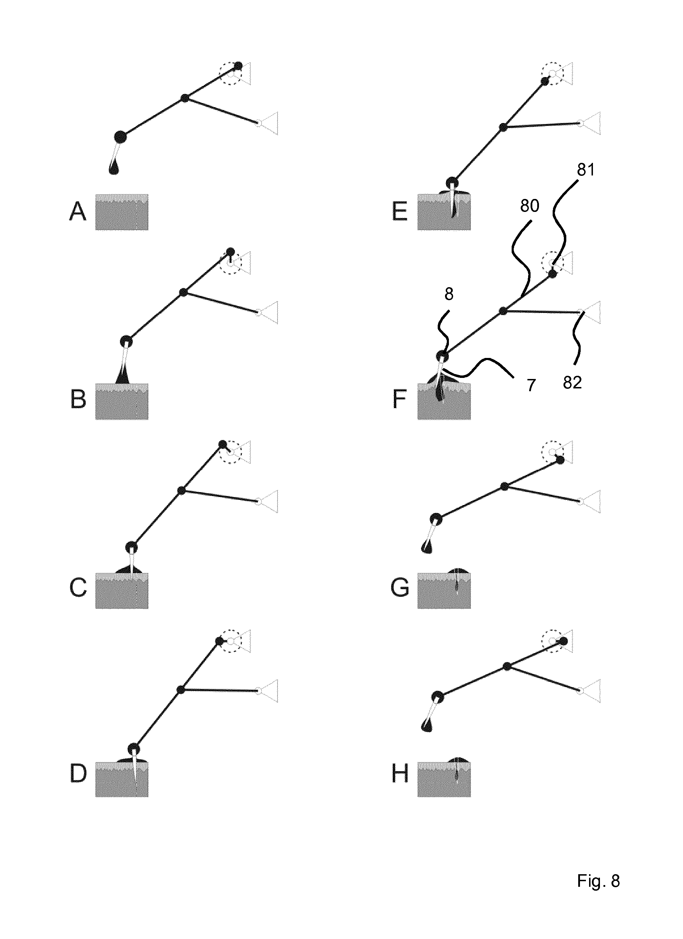

Fig. 8 zeigt eine Ausgestaltung, bei der die Stechwerkzeugaufnahme 8 an einer Gelenkanordnung 80 aufgenommen ist, die in zwei Verlagerungspunkten 81, 82 gelagert ist.

Die in der vorstehenden Beschreibung, den Ansprüchen sowie der Zeichnung offenbarten Merkmale können sowohl einzeln als auch in beliebiger Kombination für die Verwirklichung der verschiedenen Ausführungen von Bedeutung sein.

## Patentansprüche

1. Handgerät zum lokalen Aufstechen einer menschlichen oder einer tierischen Haut, bei dem in einem Gehäuse angeordnet sind
- eine Antriebseinrichtung, die eingerichtet ist, eine Antriebskraft bereitzustellen,
- eine Stecheinrichtung, bei der ein Stechwerkzeug an einer verlagerbaren Werkzeugaufnahme angeordnet ist, wobei die Werkzeugaufnahme mit dem Stechwerkzeug in Bezug auf eine Stechwerkzeugöffnung an dem Gehäuse zwischen einer eingefahrenen und einer ausgefahrenen Stellung verlagerbar ist und hierbei in einer Längsrichtung bewegt wird, und
- eine Kopplungseinrichtung, die an die Antriebseinrichtung und die Stecheinrichtung koppelt und die Antriebskraft auf die Stecheinrichtung überträgt,
wobei die Stecheinrichtung eingerichtet ist, während einer Verlagerung der Werkzeugaufnahme mit dem Stechwerkzeug zwischen der eingefahrenen und der ausgefahrenen Stellung eine durch die Antriebskraft und / oder eine weitere Antriebskraft, die im Gehäuse aktiv oder passiv erzeugt wird, erzwungene Verlagerung des Stechwerkzeugs relativ zu der Längsrichtung zuzulassen.

2. Handgerät nach Anspruch 1, **gekennzeichnet durch** einen Querbewegungsmechanismus, der an die Stecheinrichtung koppelnd in dem Gehäuse angeordnet und eingerichtet ist, eine Querbewegungskraft quer zu der Längsrichtung auf die Stecheinrichtung einzukoppeln.

3. Handgerät nach Anspruch 2, **dadurch gekennzeichnet, dass** der Querbewegungsmechanismus an die Werkzeugaufnahme koppelt und hierdurch die Querbewegungskraft wenigstens teilweise direkt in die Werkzeugaufnahme einkoppelbar ist.

4. Handgerät nach Anspruch 2 oder 3, **dadurch gekennzeichnet, dass** der Querbewegungsmechanismus direkt an das Stechwerkzeug koppelt und hierdurch die Querbewegungskraft wenigstens teilweise direkt in das Stechwerkzeug einkoppelbar ist.

5. Handgerät nach mindestens einem der Ansprüche 2 bis 4, **dadurch gekennzeichnet, dass** der Querbewegungsmechanismus eine aktive Querbewegungseinrichtung aufweist, bei der die Querbewegungskraft aktiv mittels der Antriebseinrichtung und / oder einer weiteren Antriebseinrichtung erzeugt wird, die in dem Gehäuse angeordnet ist.

6. Handgerät nach mindestens einem der Ansprüche 2 bis 5, **dadurch gekennzeichnet, dass** der Querbewegungsmechanismus eine passive Querbewegungseinrichtung aufweist, bei der die Querbewegungskraft frei von einer Antriebseinrichtung passiv erzeugt wird.

7. Handgerät nach mindestens einem der vorangehenden Ansprüche, dadurch **gekenn** - **zeichnet**, dass das Stechwerkzeug schwenkbar ist.

8. Handgerät nach mindestens einem der vorangehenden Ansprüche, **dadurch gekennzeichnet, dass** die Werkzeugaufnahme in dem Gehäuse mittels einer Führung geführt ist, die Führungsachsen in Längsrichtung und quer hierzu aufweist.

9. Handgerät nach mindestens einem der vorangehenden Ansprüche, **gekennzeichnet durch** eine Vorspanneinrichtung, mit der die Werkzeugaufnahme mit dem Stechwerkzeug gegen das Verlagern in die ausgefahrene Stellung oder das Verlagern in die eingefahrene Stellung vorgespannt ist.

10. Handgerät nach mindestens einem der vorangehenden Ansprüche, dadurch **gekenn** - **zeichnet**, dass die Werkzeugaufnahme mit einem Gelenkmechanismus verbunden ist, welcher über ein aufnahmeseitiges Gelenkelement an die Werkzeugaufnahme koppelt und bei dem das aufnahmeseitige Gelenkelement an ein oder mehrere weitere Gelenkelemente gelenkig koppelt, die an einem oder mehreren zugeordneten Gelenklagerungspunkten gelagert sind.

11. Handgerät nach mindestens einem der vorangehenden Ansprüche, **dadurch gekennzeichnet, dass** die Werkzeugaufnahme mit einer im Gehäuse aufgenommenen Dämpfungseinrichtung verbunden ist.

12. Handgerät nach mindestens einem der vorangehenden Ansprüche, **dadurch gekennzeichnet, dass** die Gehäuseöffnung eine sich quer zu der Längsrichtung erstreckenden Öffnungsführung für das Stechwerkzeug aufweist, in welcher das Stechwerkzeug infolge des Einkoppelns der Querbewegungskraft verlagert wird.

13. Verfahren zum Betrieb eines Handgeräts, die folgenden Schritte aufweisend:
- Erzeugen einer Antriebskraft mittels einer Antriebseinrichtung, die in einem Gehäuse angeordnet ist, und
- Übertragen der Antriebskraft mittels einer Kopplungseinrichtung von der Antriebseinrichtung auf eine Stecheinrichtung, bei der ein Stechwerkzeug an einer verlagerbaren Werkzeugaufnahme angeordnet ist, derart, dass die Werkzeugaufnahme mit dem Stechwerkzeug in Bezug auf eine Stechwerkzeugöffnung an dem Gehäuse zwischen einer eingefahrenen und einer ausgefahrenen Stellung verlagert wird und hierbei in einer Längsrichtung bewegt wird, und
**dadurch gekennzeichnet, dass** während einer Verlagerung der Werkzeugaufnahme mit dem Stechwerkzeug zwischen der eingefahrenen und der ausgefahrenen Stellung das Stechwerkzeug eine erzwungene Verlagerung relativ zu der Längsrichtung ausführt, wobei die Relativverlagerung durch die Antriebskraft und / oder eine weitere Antriebskraft erzwungen wird, die im Gehäuse aktiv oder passiv erzeugt wird.

## Geänderte Patentansprüche

### Geänderte Patentansprüche gemäss Regel 137(2) EPÜ.

1. Handgerät zum lokalen Aufstechen einer menschlichen oder einer tierischen Haut, bei dem in einem Gehäuse (1) angeordnet sind
- eine Antriebseinrichtung (2), die eingerichtet ist, eine Antriebskraft bereitzustellen,
- eine Stecheinrichtung, bei der ein Stechwerkzeug (7) an einer verlagerbaren Werkzeugaufnahme (8) angeordnet ist, wobei die Werkzeugaufnahme (8) mit dem Stechwerkzeug (7) in Bezug auf eine Stechwerkzeugöffnung (9) an dem Gehäuse (1) zwischen einer eingefahrenen und einer ausgefahrenen Stellung verlagerbar ist und hierbei in einer Längsrichtung bewegt wird, und
- eine Kopplungseinrichtung, die an die Antriebseinrichtung und die Stecheinrichtung koppelt und die Antriebskraft auf die Stecheinrichtung überträgt,
wobei die Stecheinrichtung eingerichtet ist, während einer Verlagerung der Werkzeugaufnahme mit dem Stechwerkzeug zwischen der eingefahrenen und der ausgefahrenen Stellung eine, erzwungene Verlagerung des Stechwerkzeugs relativ zu der Längsrichtung zuzulassen, und wobei ein Querbewegungsmechanismus (15) vorgesehen ist, der an die Stecheinrichtung koppelnd in dem Gehäuse (2) angeordnet und eingerichtet ist, eine Querbewegungskraft quer zu der Längsrichtung auf die Stecheinrichtung einzukoppeln, **dadurch gekennzeichnet, dass** der Querbewegungsmechanismus (15) eine passive Querbewegungseinrichtung aufweist, bei der die Querbewegungskraft frei von einer eigenen Antriebseinrichtung passiv im Gehäuse (1) erzeugt wird.

2. Handgerät nach Anspruch 1, **dadurch gekennzeichnet, dass** der Querbewegungsmechanismus (15) an die Werkzeugaufnahme koppelt und hierdurch die Querbewegungskraft wenigstens teilweise direkt in die Werkzeugaufnahme (8) einkoppelbar ist.

3. Handgerät nach Anspruch 1 oder 2, **dadurch gekennzeichnet, dass** der Querbewegungsmechanismus (15) direkt an das Stechwerkzeug (7) koppelt und hierdurch die Querbewegungskraft wenigstens teilweise direkt in das Stechwerkzeug (7) einkoppelbar ist.

4. Handgerät nach mindestens einem der vorangehenden Ansprüche, **dadurch gekennzeichnet,** Stecheinrichtung weiter eingerichtet ist, die Verlagerung der Werkzeugaufnahme (8) mit dem Stechwerkzeug (7) mit einem seitlichen Verschieben der Werkzeugaufhahme (8) mit dem Stechwerkzeug (7) auszuführen.

5. Handgerät nach mindestens einem der vorangehenden Ansprüche, **dadurch gekennzeichnet, dass** das Stechwerkzeug (7) schwenkbar ist.

6. Handgerät nach mindestens einem der vorangehenden Ansprüche, **dadurch gekennzeichnet, dass** die Werkzeugaufnahme (8) in dem Gehäuse (1) mittels einer Führung geführt ist, die Führungsachsen in Längsrichtung und quer hierzu aufweist.

7. Handgerät nach mindestens einem der vorangehenden Ansprüche, **gekennzeichnet durch** eine Vorspanneinrichtung, mit der die Werkzeugaufnahme (8) mit dem Stechwerkzeug (7) gegen das Verlagern in die ausgefahrene Stellung oder das Verlagern in die eingefahrene Stellung vorgespannt ist.

8. Handgerät nach mindestens einem der vorangehenden Ansprüche, **dadurch gekennzeichnet, dass** die Werkzeugaufnahme (8) mit einem Gelenkmechanismus verbunden ist, welcher über ein aufnahmeseitiges Gelenkelement an die Werkzeugaufnahme (8) koppelt und bei dem das aufnahmeseitige Gelenkelement an ein oder mehrere weitere Gelenkelemente gelenkig koppelt, die an einem oder mehreren zugeordneten Gelenklagerungspunkten gelagert sind.

9. Handgerät nach mindestens einem der vorangehenden Ansprüche, **dadurch gekennzeichnet, dass** die Werkzeugaufnahme (8) mit einer im Gehäuse (1) aufgenommenen Dämpfungseinrichtung verbunden ist.

10. Handgerät nach mindestens einem der vorangehenden Ansprüche, **dadurch gekennzeichnet, dass** die Stechwerkzeugöffnung (9) an dem Gehäuse (1) eine sich quer zu der Längsrichtung erstreckenden Öffnungsführung für das Stechwerkzeug (7) aufweist, in welcher das Stechwerkzeug infolge des Einkoppelns der Querbewegungskraft verlagert wird.

11. Verfahren zum Betrieb eines Handgeräts, die folgenden Schritte aufweisend:
- Erzeugen einer Antriebskraft mittels einer Antriebseinrichtung (2), die in einem Gehäuse (1) angeordnet ist,
- Übertragen der Antriebskraft mittels einer Kopplungseinrichtung von der Antriebseinrichtung (2) auf eine Stecheinrichtung, bei der ein Stechwerkzeug (7) an einer verlagerbaren Werkzeugaufnahme (8) angeordnet ist, derart, dass die Werkzeugaufnahme (8) mit dem Stechwerkzeug (7) in Bezug auf eine Stechwerkzeugöffnung (9) an dem Gehäuse (1) zwischen einer eingefahrenen und einer ausgefahrenen Stellung verlagert wird und hierbei in einer Längsrichtung bewegt wird, und
- Ausführen einer mittels eines Querbewegungsmechanismus (15), der an die Stecheinrichtung koppelt, erzwungenen Verlagerung des Stechwerkzeugs (7) relativ zu der Längsrichtung während der Verlagerung der Werkzeugaufnahme (8) mit dem Stechwerkzeug (7) zwischen der eingefahrenen und der ausgefahrenen Stellung,
**dadurch gekennzeichnet, dass** die Relativverlagerung durch eine weitere Antriebskraft erzwungen wird, die im Gehäuse mittels des Querbewegungsmechanismus, der frei von einer eigenen Antriebseinrichtung gebildet ist, passiv erzeugt wird.
